# EUROPÄISCHE PATENTSCHRIFT

(11) **EP 2 595 531 B1**
(45) Veröffentlichungstag und Bekanntmachung des Hinweises auf die Patenterteilung: **02.03.2022**
(21) Anmeldenummer: 11733859.0
(22) Anmeldetag: 19.07.2011
(51) Int. Cl.: A61B 5/0537, A61B 5/1172, A61B 5/00, A61G 15/02, A61M 1/16, A61M 1/14, A61G 7/075, A61G 15/12

(54) **DIALYSEEINRICHTUNG MIT EINER DIALYSEVORRICHTUNG UND EINER PATIENTENLAGERUNGSVORRICHTUNG MIT EINER EINRICHTUNG ZUR BIOIMPEDANZMESSUNG ZUR STEUERUNG UND/ODER REGELUNG DER DIALYSEVORRICHTUNG**
DIALYSIS TREATMENT SYSTEM COMPRISING A DIALYSIS DEVICE AND A PATIENT SUPPORTING DEVICE WITH A BIO-IMPEDANCE MEASURING DEVICE FOR CONTROLLING AND/OR REGULATING THE DIALYSIS DEVICE
APPAREIL DE TRAITEMENT PAR DIALYSE COMPRENANT UN DISPOSITIF DE DIALYSE ET UN SUPPORT DE PATIENT AVEC UN DISPOSITIF DE MESURE DE BIO-IMPÉDANCE POUR COMMANDER ET/OU RÉGLER LE DISPOSITIF DE DIALYSE

(30) Priorität: 19.07.2010 DE 102010031530
(43) Veröffentlichungstag der Anmeldung: 29.05.2013
(73) Patentinhaber: Fresenius Medical Care Deutschland GmbH, 61352 Bad Homburg (DE)
(72) Erfinder: CHAMNEY, Paul, Dr., Aldbury near Tring, Herts (GB); WABEL, Peter, 64287 Darmstadt (DE); MOISSL, Ulrich, 61118 Bad Vilbel (DE); WIESKOTTEN, Sebastian, 64372 Ober-Ramstadt (DE)
(74) Vertreter: Nordmeyer, Philipp Werner
(86) Internationale Anmeldenummer: PCT/EP2011/062330
(87) Internationale Veröffentlichungsnummer: WO 2012/010588

(56) Entgegenhaltungen:
- WO-A1-2006/111878
- WO-A2-2007/090119
- DE-A1-102006 032 815
- DE-U1- 29 915 914
- US-A1- 2003 216 665
- US-A1- 2004 059 242
- US-A1- 2005 031 080
- US-A1- 2005 102 165
- US-A1- 2009 275 808
- US-A1- 2010 100 003
- US-A1- 2010 168 530
- US-B1- 6 585 328

## Beschreibung

### Gebiet der Erfindung:

Die Erfindung bezieht sich auf eine Dialyseeinrichtung mit einer Dialysevorrichtung zur Durchführung der Dialysebehandlung, mit einer Patientenlagerungsvorrichtung für die Lagerung eines Patienten während einer medizinischen Dialysebehandlung, sowie mit einer Einrichtung zur Durchführung einer Bioimpedanzmessung an einem Patientenkörper zur Steuerung und/oder Regelung einer Dialysevorrichtung unter Verwendung einer solchen Patientenlagerungsvorrichtung.

### Stand der Technik:

Die Dialyse ist ein Blutreinigungsverfahren, das u.a. bei Nierenversagen als Ersatztherapie zum Einsatz kommt, und bei dem ein Stoffaustausch über eine semipermeable Membran erfolgt, wobei auf der einen Seite der Membran Patientenblut und auf der anderen Seite der Membran eine Dialyselösung (Dialysat) anliegt. Dabei sollen unerwünschte Stoffe, wie Pyrogene, Noxen, bestimmte Stoffwechselprodukte, aber auch überschüssiges Wasser durch Ultrafiltration aus dem Patientenblut entfernt werden.

Solche Dialysebehandlungen können sich über einige Stunden erstrecken, wobei der Patient während der Behandlung ruhig bzw. bewegungsarm (d.h. sitzend oder liegend) ausharren muss. Hierfür ist es seit langem im Stand der Technik üblich, Dialysestühle, -sessel oder -liegen als Patientenlagerungsvorrichtung einzusetzen, die dem Patienten eine bequeme Haltung auch während einer mehrstündigen Dialyse ermöglichen. Heute wird jedoch zumeist Patientenliegen als Lagerungsvorrichtungen der Vorzug gegeben, u.a. weil sie einen besseren Komfort als ein Sitzmöbel bieten. Bei diesen Patientenliegen ist die Patientenliegefläche im allgemeinen vielfältig verstellbar und ermöglicht neben sitzenden Positionen auch liegende Positionen des Patienten. Dadurch kann dem Patienten eine relativ komfortable Sitz-/Liegeposition während der Dialysebehandlung ermöglicht werden, die z.B. im Falle einer Hämodialysebehandlung mehrere Stunden andauert. Gleichzeitig ermöglicht eine solche Liege eine weniger kreislaufanfällige Positionierung des Patienten bei extrakorporalen Blutbehandlungen, bei denen inhärent der Blutkreislauf des Patienten beansprucht wird.

Die DE 299 15 914 U1 beschreibt eine Behandlungsliege mit einer Patientenliegefläche, die in drei Liegebereiche unterteilt ist, wobei mindestens ein Liegebereich ein Element zur Vibrationserzeugung zum Anregen des Patientenkreislaufs aufweist. Die JP 10085326 A beschreibt eine Patientenliege, in die ein Hämodialysegerät integriert ist. Auch die DE 101 41 053 B4 offenbart eine Patientenliege mit einem extrakorporalen Blutbehandlungsgerät als integralem Bestandteil. In der CH 469 490 wird ein Stuhl vorgeschlagen, in dem Teile eines Hämodialysegerätes befestigt sind. Die US 5,771,511 hat ein Krankenhausbett mit Modulen zum Gegenstand, die in ein Kommunikationsnetz eingebunden sind. Dokument US2005102165 offenbart ein System zur Kontrolle biologischer Informationen und von Informationen einer Blutbehandlungsvorrichtung mit einem Krankenbettmonitor.

Bei einer solchen Dialysebehandlung stellt die Überwachung des Hydrationsstatus des Patienten während des Dialysevorgangs einen ganz wesentlichen Faktor für Dialyseerfolg, Wohlergehen und Gesundheit des Patienten dar. Zunehmend wird hierfür eine Bioimpedanzmessung zur Bestimmung des Wassergehalts des Patienten und zur Unterstützung bei der Dialysebehandlung (d.h. insbesondere zur Festlegung der Parameter der Dialysetherapie und zur Überwachung des Behandlungserfolges) verwendet. Die Bioimpedanzmessung, oft auch in Form einer Bioimpedanzspektroskopie oder Bioimpedanz-Vektoranalyse durchgeführt, stellt ein zumeist nicht invasiv angewendetes Verfahren zur Bestimmung des intra- und extrazellulären Flüssigkeitsvolumens dar. Hierbei werden Körperimpedanzen, also Wechselstromwiderstände von Körpergeweben mittels auf der Hautoberfläche des Patienten fixierter Elektroden (Stimulationselektroden = Einkoppelelektroden; Ableitelektroden = Auskoppelelektroden) gemessen, wobei die Analyse Phasenverschiebungen und frequenzabhängige Veränderungen der Impedanz in die Analyse mit einbeziehen kann.

Beispielsweise wird im Stand der Technik bereits ein kommerziell erhältlicher Body Composition Monitor von Fresenius Medical Care AG & Co. KGaA, 61346 Bad Homburg v.d.H., Deutschland, eingesetzt, um über eine Bioimpedanzmessung den Wassergehalt in der Körperzusammensetzung des Patienten zu kontrollieren und zu Überwachung und Steuerung der Dialysevorrichtung zu nutzen. Hierfür werden Elektroden an einer Hand und einem Fuß des Patienten angebracht und durch Kabel mit dem Messgerät verbunden, wobei die gewonnenen Daten "offline" (z.B. für ärztliche Kontrolluntersuchungen) oder "online" beispielsweise zur Steuerung der Dialysevorrichtung verwendet werden können. Ein weiteres Beispiel für die Nutzung der Bioimpedanzmessung zur Bestimmung des Hydrationsstatus eines Dialysepatienten und zur Überwachung einer Dialysevorrichtung beschreibt die EP 1 645 227 B1, in welcher ein Verfahren dargestellt ist, welches den Hydrationsstatus eines Patienten durch Bioimpedanzmessung ebenfalls über an einem Gliedmaß des Patienten angelegte Elektroden bestimmt.

Da die Bioimpedanzmessung von der Körperlage beeinflusst wird, empfiehlt es sich, den Patienten vornehmlich im Liegen zu messen, da hierbei die Körperlage am ehesten reproduzierbar ist. Der Stand der Technik schlägt aber auch Apparaturen zur Messung im Sitzen vor. Die US 2004/0059242 A1 offenbart beispielsweise auf Seite 31 [FIFTH EMBODIMENT] einen Stuhl mit integrierten Elektroden zur Bestimmung der Körperzusammensetzung.

Üblicherweise kann die Messung vor und/oder nach der Behandlung stattfinden, um den Hydrationsstatus vor der Dialyse zu bestimmen, und mit dieser Kenntnis Behandlungsoptionen (Ultrafiltrationsmenge und -rate, Dialysedauer, Filtertyp, Blutfluss, Dialysatfluss, Dialysatzusammensetzung etc.) festzulegen und/oder nach der Behandlung den Therapieerfolg hinsichtlich der Entwässerung zu überprüfen. Den aus dem Stand der Technik bekannten Methoden ist gemein, dass sie einen relativ hohen personellen Aufwand durch hochqualifiziertes Personal erfordern. Zudem ist bei den meisten Geräten und Verfahren des Standes der Technik die Messgenauigkeit und/oder die Störanfälligkeit noch nicht unter allen Bedingungen zufriedenstellend gewährleistet und es kann teilweise, beispielsweise bei Patienten mit besonderen Krankheitsbildern oder außergewöhnlichen Abweichungen physiologischer Parameter, zu Fehlmessungen kommen. Darüber hinaus besteht die Möglichkeit falscher Bedienung durch das Personal bei Anlegen und Verkabelung der Elektroden für die Bioimpedanzmessung und/oder bei der Erfassung und Übertragung von Patientendaten, die für die korrekte Auswertung der Bioimpedanzmessung erforderlich sind.

### Offenbarung der Erfindung:

Es ist Aufgabe der vorliegenden Erfindung, eine Behandlungseinrichtung mit Patientenlagerungsvorrichtung und medizinischer Behandlungsvorrichtung bereitzustellen, durch die die Handhabung bei einer medizinischen Behandlung erleichtert, sowie die Einstellung und Kontrolle der medizinischen Behandlungsvorrichtung vereinfacht und die Behandlungssicherheit für den Patienten verbessert wird.

Diese Aufgabenstellung wird hinsichtlich der Dialyseeinrichtung mit einer Patientenlagerungsvorrichtung und einer Dialysevorrichtung gemäß Anspruch 1 gelöst. Einige bevorzugte Ausführungsformen sind in den abhängigen Ansprüchen ausgeführt.

Gemäß vorliegender Erfindung wird in Anspruch 1 eine Dialyseeinrichtung zur Steuerung und/oder Regelung einer Dialysevorrichtung mit einer Dialysevorrichtung zur Durchführung einer medizinischen Dialysebehandlung und einer Patientenlagerungsvorrichtung zur Lagerung eines Patienten angegeben, mit einer Einrichtung zur Durchführung einer Bioimpedanzmessung an einem Patientenkörper, wobei diese Einrichtung eine Vorrichtung zum Einkoppeln eines Messsignals in den Patientenkörper und eine Vorrichtung zum Auskoppeln einer dadurch im Patientenkörper hervorgerufenen elektrischen Messgröße aus dem Patientenkörper aufweist, und einer Schnittstelle zum Anschluss an die Dialysevorrichtung, wobei über diese Schnittstelle die mit der elektrischen Messgröße korrelierten Daten an die Dialysevorrichtung, übertragen werden können, wodurch die Dialysevorrichtung, steuerbar und/oder regelbar ist.

Das Ein- und Auskoppeln von Messsignal bzw. elektrischer Messgröße kann durch unmittelbare elektrische Kontaktierung von Hautarealen des Patienten, aber auch durch kapazitive, magnetische und/oder elektromagnetische Ein- und Auskopplung mittels Kondensatorplatten, Spulen oder anderer elektromagnetischer Abstrahlvorrichtungen erfolgen. Die Einrichtung zur Durchführung einer Bioimpedanzmessung, die in der Patientenlagerungsvorrichtung integriert ist, weist zumindest die anspruchsgegenständlichen Ein- und Auskoppelvorrichtungen eine Dialysevorrichtung, auf. Es ist möglich - und je nach Anwendungsvorgaben auch zweckmäßig -, jedoch nicht durch den Gegenstand des Anspruchs 1 vorgegeben, Signalerzeuger zur Erzeugung des Messsignals und/oder Erfassungs- und Auswertevorrichtungen für die ausgekoppelte elektrische Messgröße ebenfalls in die Patientenlagerungsvorrichtung zu integrieren und dort beispielsweise bereits die Bioimpedanz zu berechnen und über die Schnittstelle an die Dialysevorrichtung, zu übergeben oder entsprechende Steuersignale für Dialysevorrichtung, zu generieren und an diese zu übertragen. Es ist jedoch auch vom Gegenstand der Erfindung mit umfasst, dass zumindest Teile der vorgenannten Prozesse, wie beispielsweise Messsignalerzeugung und Auswertung der elektrischen Messgröße, in einer entsprechenden Verarbeitungseinheit der Dialysevorrichtung, und/oder in separaten Geräten stattfinden. Unter "mit der elektrischen Messgröße korrelierten Daten" sind alle Messwerte, Informationen und/oder Daten (und damit beispielsweise u.a. auch Bioimpedanzmessungen) zu verstehen, die unmittelbar oder mittelbar (als Rohdaten und/oder als Ergebnis eines Berechnungsprozesses unter Einbeziehung weiterer Daten) aus der elektrischen Messgröße gewonnen werden, und/oder die auf der Erregungsseite der Bioimpedanzmessung dem Messsignal zugeordnet sind. Diese Daten beinhalten wichtige physiologische und/oder pathologische Informationen über den Patienten und/oder den Dialyseverlauf (wie u.a. den Hydrationsstatus des Patienten), die über die Schnittstelle mit der Dialysevorrichtung, ausgetauscht werden können und u.a. für deren Steuerung und/oder Regelung nutzbar sind. Die einzelnen Aufbereitungs- und Verarbeitungsschritte können "vor" und/oder "hinter" der Schnittstelle in der Patientenlagerungsvorrichtung, der Dialysevorrichtung, und/oder in einem anderen Gerät vorgenommen werden.

Bei der hier angegebenen Patientenlagerungsvorrichtung ist es möglich, Ein- und Auskoppelvorrichtungen separat auszugestalten oder kombinierte Ein- und Auskoppelvorrichtungen vorzusehen. Vorzugsweise kann ein Messsignal mit mindestens zwei, vorzugsweise mit mindestens drei Frequenzen, besonders vorzugsweise mit einem kontinuierlichen Frequenzspektrum zum Einsatz kommen. Diese Frequenzen können nacheinander (sequentiell) appliziert und durchgemessen werden, aber auch simultan als komplexe periodische Signalform oder als Frequenzgemisch, dessen Fourierspektrum eine Mehrzahl von Frequenzen beinhaltet.

Nachfolgend soll die Erfindung anhand des Beispiels einer Dialysetherapie und einer Dialysevorrichtung beschrieben werden. Vorteile der erfindungsgemäßen Vorrichtungen liegen u.a. darin, dass die Handhabung bei der Durchführung der Dialysetherapie erheblich vereinfacht und beschleunigt, sowie die Fehleranfälligkeit (u.a. durch Entfall einer Patientenverkabelung) reduziert wird, so dass die Vornahme und Überwachung der Dialysebehandlung auch durch weniger qualifiziertes Personal vorgenommen werden kann. Gleichzeitig ist die Gefahr von Fehleinstellungen an der Dialysevorrichtung verringert und die Eingabe oder Übertragung von Patientendaten (wie Patientengröße, Patientengewicht, die für die korrekte Auswertung der Bioimpedanzmessung erforderlich sind; Therapieempfehlungen oder Vorgaben für die Einstellungen der Parameter an der Dialysevorrichtung durch den behandelnden Arzt) vereinfacht oder gar obsolet.

Zudem ist eine zuverlässige, kontinuierliche (oder quasi-kontinuierliche), d.h. intradialytische Überwachung, Steuerung und/oder Regelung der Dialysevorrichtung möglich, ebenso wie das Auslösen eines Alarmsignals bei Fehlfunktion oder Überschreitung bestimmter Grenzwerte. Außerdem ist durch die erfindungsgemäßen Vorrichtungen eine präzisere Erfassung des außerordentlich wichtigen Hydrationsstatus des Patienten und der dialytischen Wasseraustauschprozesse "online", also intradialytisch, während der Dialysebehandlung möglich. Die erfindungsgemäßen Vorrichtungen liefern dabei unmittelbare Aussagen über den gesamten Hydrationsstatus des Patienten, bei Einsatz der segmentalen Bioimpedanzspektroskopie sogar über einzelne Körpersegmente. Überdies erlaubt die hier vorgeschlagene Methode beispielsweise eine zusätzliche Überprüfung der Steuerung der Dialysevorrichtung und eine kontinuierliche Überwachung des Dialyseverlaufs.

Zudem lässt sich mit den erfindungsgemäßen Vorrichtungen eine hohe Messgenauigkeit (u.a. durch Minimierung von Artefakten, Erzielung einer hohen Spezifität der analysierten Parameter) und/oder eine geringe Störanfälligkeit unter nahezu allen Bedingungen auch beispielsweise bei Patienten mit besonderen Krankheitsbildern oder außergewöhnlichen Abweichungen physiologischer Parameter erzielen.

Das Messsignal kann ein elektrisches, magnetisches und/oder elektromagnetisches Wechselfeld sein, wobei die Frequenz des Wechselfeldes vorzugsweise veränderlich, insbesondere modulierbar ist. Durch den Einsatz verschiedener Frequenzen, vorzugsweise durch Frequenzmodulation des Wechselfeldes kann das komplexe System nach mehreren beispielsweise physiologischen und/oder pathologischen Parametern analysiert werden, wobei auch eine Impedanzspektroskopie durchgeführt werden kann, wodurch sich besonders aussagekräftige und präzise Ergebnisse erzielen lassen. Alternativ ist aber auch eine andersartige sequentielle Änderung der Frequenz des Wechselfeldes (beispielsweise sprunghafte Änderung) oder die simultane Anwendung mehrerer Frequenzen (beispielsweise als komplexe periodische Signalform oder als Frequenzgemisch, dessen Fourierspektrum eine Mehrzahl von Frequenzen beinhaltet) denkbar.

Daneben ist es auch möglich, das Einkoppeln des elektrischen Messsignals in den Patientenkörper und/oder das Auskoppeln der dadurch im Patientenkörper hervorgerufenen elektrischen Messgröße kapazitiv und induktiv vorzunehmen. Hierdurch lassen sich die Vorteile dieser beiden Kopplungsmethoden kombinieren, wobei die induktive Kopplung vorteilhafterweise für niedrige Frequenzen und die kapazitive Kopplung vorteilhafterweise aufgrund der Hochpasseigenschaften dieser Kopplungsmethode für höhere Frequenzen zum Einsatz kommen kann. Die verschiedenen Kopplungsmethoden können gleichzeitig oder nacheinander zur Messung genutzt werden, wobei es möglich ist, das eingekoppelte Messsignal und/oder die ausgekoppelte elektrische Messgröße für beide Kopplungsmethoden zusammenzuführen oder getrennt zu behandeln. Zudem sind derartige Kopplungsverfahren u.U. auch zur Messung durch die Bekleidung des Patienten hindurch geeignet, was weitere Vorteile für den Einsatz bietet.

Es ist oft vorteilhaft, die Ermittlung der Bioimpedanz bei einer Mehrzahl verschiedener Frequenzen des elektrischen Messsignals nach Amplitude und Phase, vorzugsweise mit hoher Auflösung der Amplitude und Phase, durchzuführen. Hierbei kann beispielsweise über ein zeitlich veränderliches Messsignal ein Messstrom in den Patientenkörper eingeprägt werden und der dadurch hervorgerufene Spannungsabfall im Patientenkörper gemessen werden. Dies stellt ein besonders vorteilhaftes Messverfahren insbesondere bzgl. hoher Messgenauigkeit und geringer Störanfälligkeit dar. Durch die getrennte Auswertung von Amplitudendämpfung und Phasenverschiebung kann die Messgenauigkeit noch weiter gesteigert werden. Zur Erzielung einer größtmöglichen Messgenauigkeit ist es zweckmäßig, die Amplitudendämpfung und Phasenverschiebung des elektrischen Messsignals mit hoher zeitlicher Auflösung zu erfassen. Hierdurch kann auch die Spezifität der Messergebnisse noch weiter gesteigert werden. Zu diesem Zweck kann beispielsweise ein entsprechender Netzwerkanalyzer Verwendung finden.

Zudem kann es von Vorteil sein, für die Analyse der Bioimpedanz eine Auswertung nach dem Cole-Modell vorzunehmen. Das Cole-Modell stellt eine einfache Beschreibung der ohmschen und kapazitiven Verhältnisse in Zweikompartiment-Systemen, wie beispielsweise Blutflüssigkeiten oder Körpergewebe, dar und bildet somit die elektrischen Verhältnisse leicht analysierbar mit guter Genauigkeit ab.

Aufgrund der kontinuierlichen Überwachung des Hydrationsstatus des Patienten ist es zudem vorteilhaft möglich, diesen Parameter zur Steuerung und/oder Regelung der Dialysevorrichtung zumindest mitzuverwenden, wodurch vorzugsweise der transmembranale Druck der Dialysevorrichtung gesteuert werden kann. Hierdurch ist es möglich, den gewünschten Wassergehalt direkt zu messen und beispielsweise über die Steuerung der Dialysevorrichtung optimal einzustellen. Eine entsprechende Regelung könnte beispielsweise über die Steuerung der Blut-, Dialysat- oder Ultrafiltrationspumpe der Dialysevorrichtung (über die Beeinflussung des Transmembrandrucks in der Dialysezelle) durch direkte Rückkopplung der zu regelnden Größe (nämlich Wassergehalt), durch ein Regelsystem erfolgen.

In einer bevorzugten Ausführungsform kann die Einrichtung zur Durchführung einer Bioimpedanzmessung eine Vorrichtung zur Bestimmung des Patientengewichts aufweisen. Da das Patientengewicht in die Berechnung der Bioimpedanz eingeht und gewissen Schwankungen unterliegt, die bedeutsam für die Durchführung und Auswertung der Bioimpedanzmessung sind, ist die Gewichtserfassung des Patienten vor der Bioimpedanzmessung obligatorisch. Durch Ausstattung der Patientenlagerungsvorrichtung mit einer Waage, die datentechnisch in die Einrichtung zur Durchführung einer Bioimpedanzmessung integriert ist, kann diese Messung automatisiert werden und unmittelbar in die Kalkulation der Bioimpedanz einfließen, wobei auch die Gewichtsveränderungen insbesondere durch die Entziehung von Überschusswasser aus dem Patientenkörper während der Dialyse mit berücksichtigt werden können. Eine separate Wägung des Patienten und Übertragung des ermittelten Gewichts an die Dialysevorrichtung kann so entfallen, wodurch Arbeitsaufwand und Fehlerquellen deutlich reduziert werden. Zudem liefert die Veränderung des Patientengewichts einen wertvollen Anhaltspunkt für die eingetretene Entwässerung, der zu Steuerung und/oder Regelung der Dialysevorrichtung genutzt werden kann.

In einer weiteren bevorzugten Ausführungsform kann die Einrichtung zur Durchführung einer Bioimpedanzmessung zumindest zwei Elektroden zum Kontaktieren des Patientenkörpers zum Einkoppeln des Messsignals in den Patientenkörper (= Einkoppelelektroden), vorzugsweise durch Einprägung eines Messstroms in den Patientenkörper, und zumindest zwei Elektroden zum Kontaktieren des Patientenkörpers zum Auskoppeln der durch das Messsignal im Patientenkörper hervorgerufenen elektrischen Messgröße aus dem Patientenkörper (= Auskoppelelektroden), vorzugsweise durch Abgreifen einer durch das Messsignal im Patientenkörper hervorgerufenen Potentialänderung, aufweisen. Hierdurch lässt sich auf besonders einfache Weise die Einkopplung und Auskopplung der elektrischen Größen gewährleisten. Durch die Verwendung getrennter Einkoppelelektroden und Auskoppelelektroden kann die Bioimpedanzmessung als Vierpunktmessung durchgeführt werden, wodurch sich die Messgenauigkeit erheblich erhöhen lässt. Besonders vorteilhaft ist es, das Messsignal als Stromsignal einzuprägen und die durch die Messstromeinprägung hervorgerufene Potentialänderung mittels eines hochohmigen Signalfolgers abzugreifen.

In einer weiteren bevorzugten Ausführungsform kann die Einrichtung zur Durchführung einer Bioimpedanzmessung eine Mehrzahl von Einkoppelelektroden und Auskoppelelektroden vorzugsweise jeweils mindestens eine Einkoppelelektrode und jeweils mindestens eine Auskoppelelektrode zur Kontaktierung jedes der Arme und jedes der Beine des Patienten zur vorzugsweisen Durchführung einer segmentalen Bioimpedanzmessung aufweisen. Dies besitzt den Vorteil, dass die Bioimpedanzmessung selektiv für einzelne Körperabschnitte durchgeführt werden kann, wodurch aussagekräftigere Auswertungen auf Ebene einzelner Körpersegmente ermöglicht werden. Zudem erzielt man durch diese Ausführungsform eine Redundanz bei der Erfassung der elektrischen Messgröße, wodurch sich die Störanfälligkeit vermindern und eine höhere Messgenauigkeit erreichen lässt. So ist es beispielsweise möglich, die Bioimpedanzmessung separat für den an den extrakorporalen Dialysekreislauf angeschlossenen Arm des Patienten oder auch einen Rechts-links-Vergleich zwischen den beiden Armen des Patienten durchzuführen, um hieraus beispielsweise anhand der längerfristigen Überwachung über mehrere Hämodialysebehandlungen hinweg der segmentalen Bioimpedanz des Arms mit der punktierten Fistel den Zustand der Fistel beispielsweise auf eine Stenose zu überwachen. Da ein großer Anteil des eingespeisten Messstroms in diesem Arm durch das Blut fließt, erhöht sich die Bioimpedanz bei reduziertem Blutfluss in der Fistel aufgrund einer einsetzenden Stenose. Diese Erhöhung kann durch eine längerfristige Überwachung und Auswertung der segmentalen Bioimpedanz des Arms mit der punktierten Fistel über mehrere Hämodialysebehandlungen hinweg detektiert werden.

Des Weiteren kann mittels dieser Anordnung frühzeitig die Bildung von Stenosen erkannt werden. Ebenfalls ist es möglich, die die Wasserverteilung und deren Änderung im Verlauf der Dialyse zu erkennen und so beispielsweise die Entwässerung von Beinen, Thorax und/oder Abdomen durch die Veränderung des bestimmbaren Überschusswassers in den jeweiligen Körpersegmenten zu überwachen. Dabei können die Einkoppelelektroden und Auskoppelelektroden beispielsweise im Bereich der Fersenauflage des rechten und linken Beins des Patienten, sowie im terminalen Unterarmbereich nahe den Handwurzelknochen des rechten und linken Arms des Patienten im Auflagebereich des Patienten auf der Patientenlagerungsvorrichtung angeordnet sein, wobei die Einkoppelelektroden vorteilhafterweise distal der Auskoppelelektroden vorgesehen werden, um hierdurch das Messprinzip der "Vierpunktmessung" umzusetzen. Es ist jedoch auch denkbar, die Zahl der Messstellen (= Einkoppelelektroden) zu erhöhen und diese zusätzlich im Wadenbereich des Patienten, im Lendenbereich des Patienten, im Nackenbereich des Patienten, sowie im Ellenbogenbereich des Patienten - bei lateral der Sagittalebene angeordneten Elektroden jeweils paarweise symmetrisch für beide Körperhälften - im Auflagebereich des Patienten auf der Patientenlagerungsvorrichtung anzuordnen. Hierdurch lässt sich eine sehr genaue Analyse der verschiedenen Körpersegmente bei gleichzeitig einfacher und zuverlässiger Kontaktierung gewährleisten.

In einer weiteren bevorzugten Ausführungsform kann die Bioimpedanzmessung eine Bioimpedanzspektroskopie und/oder eine Bioimpedanz-Vektoranalyse, vorzugsweise unter selektiver Erfassung von Amplitudenänderung und Phasenverschiebung der elektrischen Messgröße, besonders vorzugsweise mit hoher Auflösung, sein. Hierdurch kann eine besonders hohe Messgenauigkeit und Spezifität der Messergebnisse bei der Detektierung der verschiedenen physiologischen und/oder pathologischen Parameter gewährleistet werden.

In einer weiteren bevorzugten Ausführungsform kann die Bioimpedanzmessung eine lineare und eine nicht-lineare Analyse der elektrischen Messgröße umfassen. Durch zusätzliche Messung beispielsweise von Ein- und Ausschwingvorgängen, "white noise"-Analysen oder der dynamischen Pulsantwort der gemessenen Gewebsbereiche des Patienten können weitere, detaillierte Analysen vorgenommen und der Nachweis bzw. die Quantifizierung einer Vielzahl von physiologischen und/oder pathologischen Parameter nebeneinander zuverlässig durchgeführt werden.

In einer weiteren bevorzugten Ausführungsform kann die Messgröße der Bioimpedanzmessung am Patientenkörper gemeinsam auswertbar sein mit einer Messgröße einer Blutanalyse in einem extrakorporalen Kreislauf der Dialysevorrichtung. Durch die Kopplung von Bioimpedanzmessungen am Patientenkörper mit entsprechenden Analysen des Blutes im extrakorporalen Kreislauf der Dialysevorrichtung vor und hinter dem Dialysefilter können zahlreiche weitere Informationen gewonnen werden (so können Veränderungen in der Blutzusammensetzung der zellulären und extrazellulären Bestandteile durchgeführt und mit den Bioimpedanz-Messwerten aus dem Patientenkörper korreliert werden Hierbei kann die Blutanalyse jegliche Analyse des Blutes umfassen, z.B. Glukose-, Natrium-, Hämoglobin-, Harnstoff- oder Albuminbestimmung durch jedwede Art von Sensoren wie optische, akustische oder elektrische Sensorvorrichtungen, insbesondere Bioimpedanzmessung.

Die Patientenlagerungsvorrichtung weist eine Vorrichtung zur Bestimmung der Patientengröße mit Drucksensoren und/oder Drucksensorfeldern auf, die im Bereich von Patientenauflageflächen der Patientenlagerungsvorrichtung angeordnet sind. Vorteilhafterweise kann auch die zur Auswertung der Bioimpedanzmessung erforderliche Größenangabe des Patienten automatisch beispielsweise mittels Druck-, Kontakt- und/oder Näherungssensoren ermittelt werden. Auch ist es denkbar hierfür Ultraschall- oder Lasermessgeräte zur Distanzmessung beispielsweise zu Fußsohlen und Scheitel zu verwenden, um die Körpergröße des Patienten zu bestimmen.

Die Patientenlagerungsvorrichtung kann zur Bestimmung der Patientengröße Sensoren umfassen, die die Stellung einer an der Patientenlagerungsvorrichtung angebrachten Fußstütze ermitteln. Es hat sich gezeigt, dass die Position der Fußstütze mit der Körpergröße des Patienten gut korreliert, sodass aus der bekannten Position der Fußstütze die Körpergröße abgeleitet werden kann.

In einer weiteren bevorzugten Ausführungsform kann die Patientenlagerungsvorrichtung eine optische Messvorrichtung zur vorzugsweise mehrdimensionalen, insbesondere dreidimensionalen Erfassung der Patientenmaße aufweisen. Dabei können beispielsweise eine oder mehrere Laserscanner, Kameras und/oder 3D-Scanner eingesetzt werden, um beispielsweise die Körpergröße oder andere wichtige Körperabmessungen, ein zweidimensionales Körperprofil beispielsweise in Sagittalebene (z.B. zur Verbesserung der Messgenauigkeit der nachstehend beschriebenen Berechnung des hydrostatischen Druckes), oder auch dreidimensionale Modelle der Körperoberfläche zu ermitteln, beispielsweise auch um mögliche Atem- oder Bewegungsartefakte bei den Messungen oder Volumenänderung (z.B. von Beinen und/oder Abdomen) durch die dialytische Entwässerung zu erfassen.

In einer weiteren bevorzugten Ausführungsform kann die Patientenlagerungsvorrichtung eine Einrichtung zur Erfassung und/oder Berechnung des hydrostatischen Druckes zumindest unter Verwendung von Lage-Einstellungsparametern der Patientenlagerungsvorrichtung, insbesondere von Neigung der Rückenlehne und/oder Fußstütze, aufweisen. Im Stand der Technik werden Verfahren zur Messung des arteriellen Flusses beschrieben. Die US 7,172,569 offenbart beispielsweise ein Verfahren, das hierzu den venösen und den arteriellen Druck bei Variation des Blutflusses misst. Wesentlich hierbei ist, dass der Patient seine Position während der Messung nicht verändert, da insbesondere die Armposition direkte Auswirkungen auf die Drucksignale hat. Ist nun die Position der Rückenlehne durch die Lehneneinstellung der Patientenlagerungsvorrichtung bekannt, und ist überdies durch die plausible Messung der segmentalen Bioimpedanz sichergestellt, dass der Arm auch auf dieser Lehne ruht, kann hierdurch der hydrostatische Druck abgeschätzt werden. Durch Kombination mit der zuvor genannten mehrdimensionalen Erfassung des Patientenkörpers kann die Genauigkeit dieser Schätzung noch deutlich verbessert werden.

In einer weiteren bevorzugten Ausführungsform kann die Patientenlagerungsvorrichtung eine Einrichtung zur Erfassung der Positionen der Armlehnen umfassen. Zur Bestimmung des Blutdrucks in der Fistel des Patienten kann die Dialysemaschine mit einem Drucksensor ausgestattet sein. Dieser Drucksensor kann aber nur den Druck im Blutschlauch zur Fistel bestimmen. Die hydrostatische Druckdifferenz zwischen der Höhe des Drucksensors an der Dialysemaschine und der Höhe des Patientenarms geht in die Berechnung des Fisteldrucks ein. Durch die Kenntnis der Lage des Patientenarms, insbesondere der Höhe gegenüber dem Boden, ist die Höhe der Fistel weitgehend bestimmbar, und damit kann die hydrostatische Druckdifferenz bestimmt werden.

In einer weiteren bevorzugten Ausführungsform kann die Patientenlagerungsvorrichtung eine motorische Verstellvorrichtung vorzugsweise von zumindest Rückenlehne und/oder Fußstütze aufweisen. Besonders bevorzugt ist eine motorische Verstellvorrichtung, die durch die Dialysevorrichtung steuerbar ist.

Hierdurch kann einerseits eine bequeme und für die Dialyse und die geplanten Messungen vorteilhafte Positionierung des Patientenkörpers vorgenommen werden. Zudem ist es möglich, eine weniger kreislaufanfällige Positionierung des Patienten bei extrakorporalen Blutbehandlungen, bei denen inhärent der Blutkreislauf des Patienten beansprucht wird, einzustellen oder im Bedarfsfall einem kritischen Kreislaufzustand des Patienten entgegenzuwirken. Es ist zudem auch aus dem Stand der Technik bekannt, dass die Patientenhaltung, oder die Lage des Patienten auf der Patientenlagerungsvorrichtung die Bioimpedanzmessung beeinflusst. Durch die motorisierte Verstellbarkeit der Patientenlagerungsvorrichtung kann einerseits der Einfluss der dann im Wesentlichen bekannten Patientenposition evaluiert werden (in den Körperlagen aktiv durch Lehnenverstellung "erzwungen" werden) und andererseits die durch die Einstellung der Patientenlagerungsvorrichtung bekannte Körperlage mit in die Bestimmung der Bioimpedanz einfließen. Weiterhin kann die motorisierte Verstellbarkeit dazu genutzt werden, die orthostatische Regulation des Patientenkreislaufs zu evaluieren und zu beeinflussen, aber auch dazu, die Messung des hydrostatischen Drucks zu verbessern, indem die Einstellung von Rückenlehne, Fußstütze und Armlehnen während dieser Messung in eine Optimalposition bewegt wird, oder indem die Messung des hydrostatischen Drucks bei verschiedenen Positionen vorgenommen wird, wodurch sich die Messgenauigkeit für den hydrostatischen Druck weiter steigern lässt. Des Weiteren kann der Zustand des Patienten, beispielsweise durch die Dialysevorrichtung, überwacht werden. Falls ein Kollaps des Patienten erkannt wird oder dessen Kreislaufparameter kritische Werte annehmen oder anzunehmen drohen, kann die Dialysevorrichtung die motorische Verstellvorrichtung der Patientenlagerungsvorrichtung derart ansteuern, dass der Patient in eine Schockposition, beispielsweise in die Trendelenburg-Position, gebracht wird.

In einer weiteren bevorzugten Ausführungsform kann die Patientenlagerungsvorrichtung eine Vorrichtung zur Feststellung der Patientenidentität, vorzugsweise mit einem Fingerabdrucksensor, aufweisen. Dies kann beispielsweise durch Einsatz einer Patientenkarte (Smartcard mit einem Speicherchip beispielsweise der behandeln Einrichtung, Chipkarte der Krankenversicherung, oder eine RFID-Karte) oder beispielsweise auch mit einem Barcode auf einer Patientenkarteikarte erfolgen. Mit Vorteil kann hierfür jedoch auch ein Fingerabdrucksensor, beispielsweise integriert in die Armlehne der Patientenlagerungsvorrichtung zum Einsatz kommen. Durch einen solchen Sensor kann der Patient eindeutig identifiziert und somit zuverlässig auf einen individuellen gespeicherten Datensatz dieses Patienten zurückgegriffen werden. Dieser kann beispielsweise in der Dialysevorrichtung selbst gespeichert sein, oder vorteilhafterweise auf einem vernetzten Server vorgehalten werden.

Die Erfindung wird nicht durch die konkreten Ausführungsformen begrenzt; die Merkmale aller vorgenannten Ausführungsformen sind - soweit sie sich nicht gegenseitig technisch ausschließen oder negativ beeinträchtigen - frei miteinander kombinierbar.

### Beschreibung der Figuren:

Anhand der Zeichnungen werden zwei Ausführungsbeispiele der Erfindung nachstehend eingehend erläutert. Es zeigen:
- Fig. 1: Ersatzschaltbild für die segmentalen Bioimpedanzen des menschlichen Körpers;
- Fig. 2: Bioimpedanzen des menschlichen Körpers (rechte Seite Messkurven - linke Seite kalkulierte Bioimpedanzen der Extremitäten);
- Fig. 3: eine Ausführungsform einer erfindungsgemäßen Patientenlagerungsvorrichtung mit einer Anzeige- und Bedienvorrichtung für die Positionseinstellung und Bioimpedanzmessung; und
- Fig. 4: eine Ausführungsform einer erfindungsgemäßen Patientenlagerungsvorrichtung, die mit einer Dialysevorrichtung verbunden ist.

Die Bioimpedanzmessung, wie sie im Detail in der deutschen Patentanmeldung DE 10 2010 028 902.7 beschrieben ist, kann in menschlichen Geweben und auch nicht-invasiv am Körper des Patienten durchgeführt werden. Dazu können beispielsweise jeweils eine Einkoppelelektrode und eine Auskoppelelektrode mit den Endabschnitten zweier Extremitäten (also beispielsweise rechter Arm und linker Arm oder rechtes Bein und linkes Bein oder rechter Arm und rechtes Bein) verbunden werden. Dabei sollte sich an der entsprechenden Extremität die Einkoppelelektrode jeweils distal der Auskoppelelektrode befinden, um das Anordnungsprinzip der "Vierpunktmessung" zu nutzen. Im Folgenden schließt die Bezeichnung linker bzw. rechter Arm immer auch die jeweils zugehörige Hand ein und die Bezeichnung linkes bzw. rechtes Bein immer auch den jeweils zugehörigen Fuß ein.

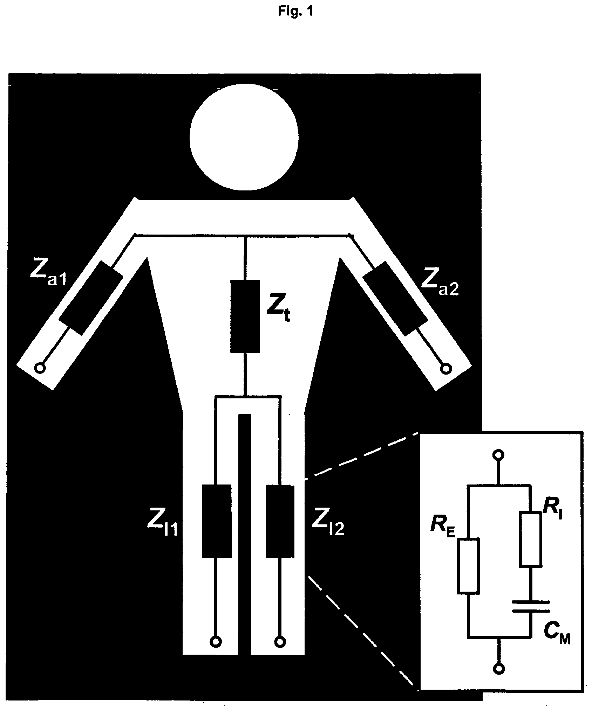

Besonders vorteilhaft ist jedoch eine segmentale Bioimpedanzmessung mit acht Elektroden, bei der an jedem Arm und jedem Bein des Patienten jeweils Einkoppelelektroden und Auskoppelelektroden nach dem zuvor genannten Prinzip angeordnet sind. Dadurch ist es möglich, die Bioimpedanz der einzelnen Arme und Beine, sowie des Rumpfes des Patienten zu ermitteln. Das Prinzip wird anhand der Fig. 1 erläutert
Aus den sechs Messungen:
   - Z₁: Rechte Körperseite
   - Z₂: Linke Körperseite
   - Z₃: Rechte Hand → linker Fuß
   - Z₄: Linke Hand → rechter Fuß
   - Z₅: Rechte Hand → linke Hand
   - Z₆: Rechter Fuß → linker Fuß
können aus dem in Fig. 1 dargestellten Widerstandsnetzwerks des Ersatzschaltbildes für die Körperimpedanzen des Patienten die fünf segmentalen Bioimpedanzen:
   - Zₐ₁: Rechter Arm
   - Zₐ₂: Linker Arm
   - Zₜ: Rumpf
   - Zₗ₁: Rechtes Bein
   - Zₗ₂: Linkes Bein
errechnet werden.

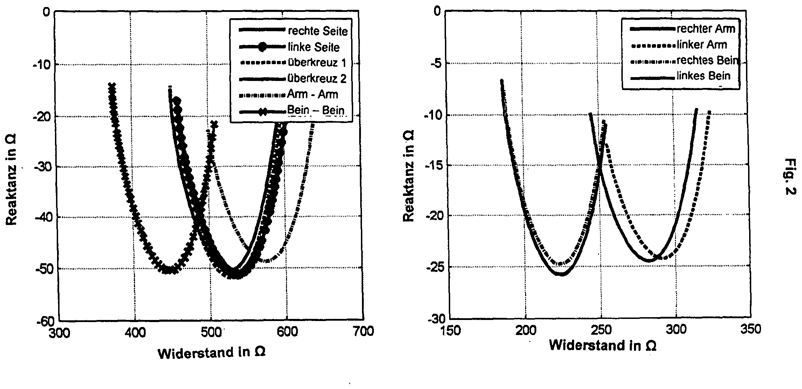

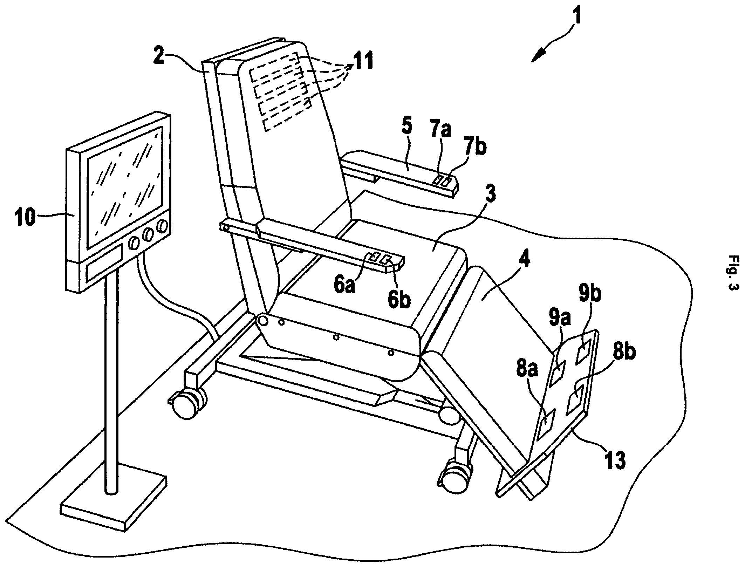

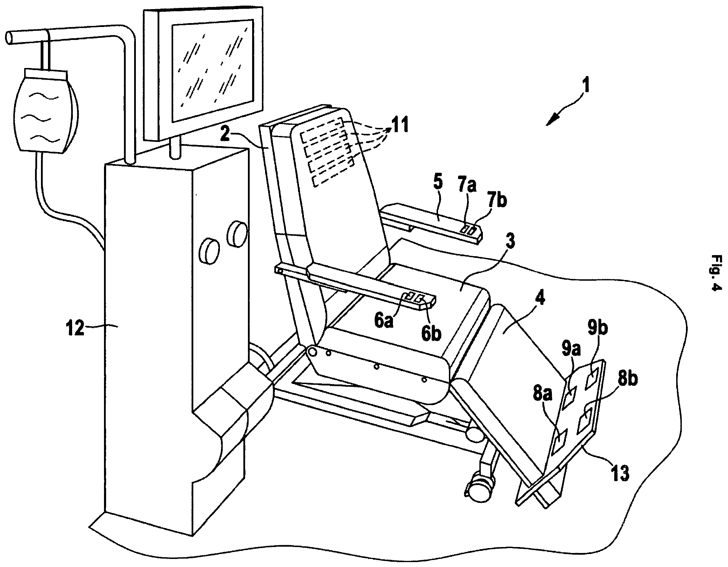

Hierbei erhält man sechs Messkurven für die Körperimpedanzen aus den Bioimpedanzmessungen, wie sie im linken Diagramm von Fig. 2 dargestellt sind. Durch Berechnung des in Fig. 1 dargestellten Widerstandsnetzwerks aus dem Ersatzschaltbildes für die Körperimpedanzen des Patienten lassen sich so beispielsweise die fünf Bioimpedanzkurven für die einzelnen Körpersegmente der Arme und Beine des Patienten ermitteln (Fig. 2 rechts). Hierdurch lassen sich - einerseits durch Redundanz und andererseits durch Signalanalyseverfahren - die Störanfälligkeiten vermindern und höhere Messgenauigkeiten erreichen, sowie aussagekräftigere Auswertungen auf Ebene einzelner Körpersegmente vornehmen. Deshalb wird die vorgenannte Anordnung mit den acht Elektroden vorzugsweise in der im Folgenden beschriebenen und in den Figuren 3 und 4 dargestellten Patientenlagerungsvorrichtung eingesetzt.

Es ist ebenso möglich, dass Ein- und Auskoppelsensoren einer Messung unterschiedlichen Körperteilen zugeordnet sind. So kann beispielsweise eine Stromeinprägung von der rechten Hand zum linken Fuß erfolgen und ein Spannung zwischen rechtem und linkem Fuß gemessen werden. Auf diese Weise wird nur die Bioimpedanz des rechten Beins bestimmt.

Die Patientenlagerungsvorrichtung umfasst eine Patientenliege (1), wie sie aus dem Stand der Technik grundsätzlich bekannt ist, mit einer Patientenliegefläche, deren Rückenlehne (2), ggf. Kopfstütze, Sitzfläche (3) und Beinablage (4) vielfältig verstellbar ist, sowie mit verstellbaren Armablagen (5), und ermöglicht neben sitzenden Positionen auch liegende Positionen des Patienten.

Die Patientenlagerungsvorrichtung weist eine Patientenliegefläche auf, die in drei Segmente (Rückenlehne (2), Sitzfläche (3) und Beinablage (4)) unterteilt ist. Zwischen den einzelnen Segmenten befinden sich Drehgelenke, die mittels motorischer Stelleinrichtungen verstellbar sind, so dass die drei Segmente in vielfältiger Weise zueinander verschwenkt werden können. Weiter kann auch noch eine motorisch verstellbare Kopfstütze vorgesehen sein, um dem Patienten eine angenehmere Lagerung des Kopfes bei Einstellung der Patientenlagerungsvorrichtung in einer Liegeposition zu ermöglichen. Außerdem ist es möglich, die Patientenlagerungsvorrichtung mit motorisch höhenverstellbaren Armstützen (5) auszustatten, um hierdurch den hydrostatischen Druck noch besser beeinflussen zu können. Zusätzlich ist unterhalb des Sitzflächensegmentes eine Hub- und Schwenkeinheit versehen, mit der die Höhe und der Neigungswinkel dieses Segmentes eingestellt werden können. Die Bedienung der Einheit erfolgt wahlweise über eine manuelle Einstelleinheit (10) oder gesteuert von der Dialysevorrichtung (12).

Die Patientenlagerungsvorrichtung weist als Einkoppelelektroden (6a, 7a, 8a, 9a) und Auskoppelelektroden (6b, 7b, 8b, 9b) in der Fußstütze (13), die in ihrer Position an die Beinlänge des Patienten anpassbar sein kann, im Bereich der Fersenauflage des rechten und des linken Beins des Patienten, sowie in den Armauflagen (5) im terminalen Unterarmbereich nahe den Handwurzelknochen des rechten und des linken Arms des Patienten im jeweiligen Auflagebereich des Patienten auf der Patientenlagerungsvorrichtung Elektrodenfelder (lineare Kontaktelektrodenarrays) auf, die nach Platzierung des Patienten auf der Patientenliegefläche der Patientenlagerungsvorrichtung von den entsprechenden, nicht mit Kleidungsstücken bedeckten Hautarealen elektrisch kontaktiert werden und die Einkoppelelektroden (6a, 7a, 8a, 9a) und Auskoppelelektroden (6b, 7b, 8b, 9b) aufweisen; zusätzlich kann eine Fixierung durch Gurtbänder vorgesehen sein, um im Bedarfsfall den Patientenkörper so zu fixieren, dass die Kontaktierung während der Behandlungsdauer sichergestellt werden kann (kurzfristige Patientenbewegungen, die zu einer temporären Unterbrechung des elektrischen Kontakts führen, stellen jedoch kein Problem dar). Es ist jedoch auch denkbar, die Zahl der Messstellen (= Einkoppelelektroden) zu erhöhen und diese zusätzlich im Wadenbereich des Patienten, im Lendenbereich des Patienten, im Nackenbereich des Patienten, sowie im Ellenbogenbereich des Patienten - bei lateral der Sagittalebene angeordneten Elektroden jeweils paarweise symmetrisch für beide Körperhälften - im Auflagebereich des Patienten auf der Patientenlagerungsvorrichtung anzuordnen.

Die Elektroden bzw. Elektrodenfelder können mit einem elastisch erhabenen (oder federnden) Anlagebereich versehen sein, um die Kontaktierungssicherheit mit dem Hautareal des Patienten zu verbessern. Innerhalb der einzelnen Elektrodenfelder wird jeweils nur diejenige Elektrode durchgeschaltet, die der anatomischen Sollposition am nächsten kommt. Dies kann beispielsweise dadurch gewährleistet werden, dass der Patient automatisch vermessen wird (s.u.) und die Sollpositionen der Elektroden aus dieser Messung errechnet werden, oder dadurch, dass durch elektrische Messungen die jeweils bestplatzierte Elektrode (z.B. als die jeweils äußerste kontaktierte Elektrode) ermittelt wird oder dadurch, dass beispielsweise Drucksensoren die Auflagekraft im jeweiligen Elektrodenbereich (integriert über einen längeren Messzeitraum, um Verfälschungen durch Bewegungsartefakte zu minimieren) messen und hierdurch beispielsweise die Körpergröße und die jeweils zu verwendende Elektrode aus der Körpergröße bestimmt werden.

Weiterhin besitzt die Patientenlagerungsvorrichtung eine Vorrichtung zur Bestimmung des Patientengewichts, das automatisch ermittelt und bei den Berechungen beispielsweise der Bioimpedanzmessung und bei der Einstellung der Dialysevorrichtung verwendet werden kann, wodurch eine vorab durchgeführte Wägung mit manueller Gewichtseingabe entfallen kann. Die Wägung kann dadurch erfolgen, dass das Gewicht der Patientenliegefläche einschließlich des darauf befindlichen Patienten ermittelt wird und von diesem das (bekannte) Gewicht der leeren Patientenliegefläche subtrahiert wird. Das Ergebnis der Wägung kann im Display der Einstelleinheit (10) bzw. der Dialysevorrichtung (12) angezeigt werden.

Des Weiteren befinden sich in die Rückenlehne integriert mehrere Drucksensoren (11), um die Größe des Patienten bestimmen zu können. Alternativ befindet sich an einem Ausleger seitlich und oberhalb des Patienten, oder wahlweise an Decke und Wänden des Behandlungsraumes zwei Laser-Scanner, mittels derer die Körperoberfläche des Patienten abgetastet und dreidimensional über den gesamten Behandlungszeitraum erfasst werden kann. Hieraus lassen sich Körpergröße des Patienten und Statur bzw. Körperbau (beispielsweise für die präzise Berechnung des hydrostatischen Drucks) bestimmen, aber auch Atem- und Bewegungsartefakte erkennen und Volumenänderungen beispielsweise im Bereich der Beine, des Thorax oder des Abdomens durch die dialytische Entwässerung erkennen. Es ist jedoch auch möglich und in vielen Fällen hinreichend, lediglich die Körpergröße mittels der zuvor erwähnten Drucksensoren, oder mittels Ultraschall- oder Laser-Entfernungsmessung (durch Distanzmessung auf Scheitel und Fußsohlen) zu erfassen.

Außerdem kann die Patientenlagerungsvorrichtung noch einen Fingerabdruckssensor im Fingerauflagebereich der Armlehnen besitzen, mittels dessen eine eindeutige Patientenidentifizierung vorgenommen und der automatische Abruf des Patientendatensatzes mit allen relevanten abgespeicherten Informationen über den Patienten und über die Daten der Dialysebehandlung beispielsweise von einem PC oder einem Krankenhaus-Server sichergestellt werden kann.

Die Einkoppelelektroden (6a, 7a, 8a, 9a) sind mit einem Messsignalerzeuger, dessen Messsignal über die Einkoppelelektroden in den Körper des Patienten eingekoppelt wird, elektrisch verbunden. Die Auskoppelelektroden (6b, 7b, 8b, 9b), durch welche die durch das eingekoppelte Messsignal (ein im Frequenzbereich von beispielsweise 1 kHz bis 1 MHz frequenzmodulierter, eingeprägter Messstrom) im Patientenkörper hervorgerufene elektrische Messgröße ausgekoppelt wird, sind elektrisch mit einer Erfassungseinrichtung verbunden, mittels derer die ausgekoppelte elektrische Messgröße erfasst und durch die Auswerteeinrichtung (eigenständig (10) oder in die Dialysevorrichtung (12) integriert) mittels Auswertung und Berechnung über den Frequenzbereich verarbeitet wird.

So kann hieraus beispielsweise der Hydrationsstatus des Patienten und das dialytisch zu entfernende Überschuss-Körperwasser bestimmt werden. Über eine entsprechende Schnittstelle zum Anschluss der Dialysevorrichtung (12) wird dann eine entsprechende Information an die Dialysevorrichtung (12) übermittelt, die wiederum diese Information, beispielsweise zur Einstellung des transmembranalen Druckes in der Dialysekammer des Dialysators, über entsprechende Ansteuerung der Blut-, Dialysat- oder Ultrafiltrationspumpen der Dialysevorrichtung (12) heranziehen kann.

### Bezugszeichenliste

- 1: Patientenliege
- 2: Rückenlehne
- 3: Sitzfläche
- 4: Beinablage
- 5: Armlehne
- 6a, 6b: Elektroden, rechte Hand
- 7a, 7b: Elektroden, linke Hand
- 8a, 8b: Elektroden, rechter Fuß
- 9a, 9b: Elektroden, linker Fuß
- 10: Anzeige- und Bedienvorrichtung für Positionseinstellung und Bioimpedanzmessung
- 11: Drucksensoren
- 12: Dialysevorrichtung
- 13: Fußstütze

## Patentansprüche

1. Dialyseeinrichtung zur Steuerung und/oder Regelung einer Dialysevorrichtung, aufweisend:
- eine Dialysevorrichtung (12) zur Durchführung einer medizinischen Dialysebehandlung
- eine Patientenlagerungsvorrichtung (1) zur Lagerung eines Patienten mit einer Einrichtung zur Durchführung einer Bioimpedanzmessung an einem Patientenkörper, wobei diese Einrichtung
- eine Vorrichtung (6a, 7a, 8a, 9a) zum Einkoppeln eines Messsignals in den Patientenkörper und
- eine Vorrichtung (6b, 7b, 8b, 9b) zum Auskoppeln einer dadurch im Patientenkörper
hervorgerufenen elektrischen Messgröße aus dem Patientenkörper aufweist, und
einer Schnittstelle zum Anschluss an die Dialysevorrichtung (12) wobei die Patientenlagerungsvorrichtung(1) eine Vorrichtung zur Ermittlung der Patientengröße mit Drucksensoren (11) und/oder Drucksensorfeldern aufweist, die im Bereich von Patientenauflageflächen der Patientenlagerungsvorrichtung (1) angeordnet sind, und
- eine Auswerteeinrichtung zur Auswertung der durch die Einrichtung zur Durchführung einer Bioimpedanzmessung ausgekoppelten elektrischen Messgröße unter Berücksichtigung der ermittelten Patientengröße und zur Übertragung der mit der ausgewerteten elektrischen Messgröße korrelierten Daten an die Dialysevorrichtung (12) über die Schnittstelle, wobei die Dialysevorrichtung (12) derart eingerichtet ist, durch die mit der ausgewerteten elektrischen Messgröße korrelierten Daten gesteuert und/oder geregelt zu werden.

2. Dialyseeinrichtung nach Anspruch 1, **dadurch gekennzeichnet, dass** die Einrichtung zur Durchführung einer Bioimpedanzmessung eine Vorrichtung zur Bestimmung des Patientengewichts aufweist.

3. Dialyseeinrichtung nach einem der vorhergehenden Ansprüche, **dadurch gekennzeichnet, dass** die Einrichtung zur Durchführung einer Bioimpedanzmessung zumindest zwei Elektroden zum Kontaktieren des Patientenkörpers zum Einkoppeln des Messsignals in den Patientenkörper (= Einkoppelelektroden), vorzugsweise durch Einprägung eines Messstroms in den Patientenkörper, und
zumindest zwei Elektroden zum Kontaktieren des Patientenkörpers zum Auskoppeln der durch das Messsignal im Patientenkörper hervorgerufenen elektrischen Messgröße aus dem Patientenkörper (= Auskoppelelektroden), vorzugsweise durch Abgreifen einer durch das Messsignal im Patientenkörper hervorgerufenen Potentialänderung, aufweist.

4. Dialyseeinrichtung nach einem der vorhergehenden Ansprüche, **dadurch gekennzeichnet, dass** die Einrichtung zur Durchführung einer Bioimpedanzmessung eine Mehrzahl von Einkoppelelektroden und Auskoppelelektroden vorzugsweise jeweils mindestens eine Einkoppelelektrode und jeweils mindestens eine Auskoppelelektrode zur Kontaktierung jedes der Arme und jedes der Beine des Patienten zur vorzugsweisen Durchführung einer segmentalen Bioimpedanzmessung aufweist.

5. Dialyseeinrichtung nach einem der vorhergehenden Ansprüche, **dadurch gekennzeichnet, dass** die elektrische Messgröße der Bioimpedanzmessung am Patientenkörper gemeinsam auswertbar ist mit einer Blutanalyse in einem extrakorporalen Kreislauf der Dialysevorrichtung.

6. Dialyseeinrichtung nach einem der vorhergehenden Ansprüche, **dadurch gekennzeichnet, dass** die Patientenlagerungsvorrichtung eine optische Messvorrichtung zur vorzugsweise mehrdimensionalen, insbesondere dreidimensionalen Erfassung der Patientenmaße aufweist.

7. Dialyseeinrichtung nach einem der vorhergehenden Ansprüche, **dadurch gekennzeichnet, dass** die Patientenlagerungsvorrichtung eine Einrichtung zur Erfassung und/oder Berechnung des hydrostatischen Druckes zumindest unter Verwendung von Lage-Einstellungsparametern der Patientenlagerungsvorrichtung, insbesondere von Neigung der Rückenlehne und/oder Beinablage und/oder Fußstütze, aufweist, und/oder wobei die Patientenlagerungsvorrichtung eine Einrichtung zur Erfassung der Positionen von Armlehnen der Patientenlagerungsvorrichtung aufweist.

8. Dialyseeinrichtung nach einem der vorhergehenden Ansprüche, **dadurch gekennzeichnet, dass** die Patientenlagerungsvorrichtung eine motorische Verstellvorrichtung vorzugsweise von zumindest Rückenlehne und/oder Beinablage und/oder Fußstütze aufweist, wobei die motorische Verstellvorrichtung bevorzugt durch die Dialysevorrichtung steuerbar ist.

9. Dialyseeinrichtung nach einem der vorhergehenden Ansprüche, **dadurch gekennzeichnet, dass** die Patientenlagerungsvorrichtung eine Vorrichtung zur Feststellung der Patientenidentität, vorzugsweise mit einem Fingerabdrucksensor, aufweist.

## Claims

1. Dialysis treatment arrangement for controlling and/or regulating a dialysis device, comprising:
- a dialysis device (12) for carrying out a medical dialysis treatment,
- a patient supporting device (1) for supporting a patient with an arrangement for carrying out a bio-impedance measurement on a patient's body, wherein this arrangement comprises
- a device (6a, 7a, 8a, 9a) for coupling in a measurement signal into the patient's body and
- a device (6b, 7b, 8b, 9b) for decoupling an electrical measurement variable, generated thereby in the patient's body, out of the patient's body, and an interface for connection to the dialysis device (12)
wherein the patient supporting device (1) comprises a device for determining the patient's size with pressure sensors (11) and/or pressure sensor fields, which are arranged in the region of patient supporting surfaces of the patient supporting device (1), and
- an evaluation arrangement for evaluating the electrical measurement variable which has been decoupled by the arrangement for carrying out a bio-impedance measurement, taking into account the determined patient's size, and for transmitting the data correlated with the evaluated electrical measurement variable to the dialysis device (12) via the interface,
wherein the dialysis device (12) is configured to be controlled and/or regulated by the data correlated with the evaluated electrical measurement variable.

2. Dialysis arrangement according to claim 1, **characterized in that** the arrangement for carrying out a bio-impedance measurement comprises a device for determining the patient's weight.

3. Dialysis arrangement according to one of the preceding claims, **characterized in that** the arrangement for carrying out a bio-impedance measurement comprises at least two electrodes for contacting the patient's body to couple the measurement signal into the patient's body (= coupling electrodes), preferably by introducing a measuring current into the patient's body, and
at least two electrodes for contacting the patient's body for decoupling the electrical measurement variable, generated by the measurement signal in the patient's body, out of the patient's body (= decoupling electrodes), preferably by tapping a potential change generated by the measurement signal in the patient's body.

4. Dialysis arrangement according to one of the preceding claims, **characterized in that** the arrangement for carrying out a bio-impedance measurement comprises a plurality of coupling electrodes and decoupling electrodes preferably at least one respective coupling electrode and at least one respective decoupling electrode for contacting each of the arms and each of the legs of the patient for the preferable conducting of a segmental bio-impedance measurement.

5. Dialysis arrangement according to one of the preceding claims, **characterized in that** the electrical measurement variable of the bio-impedance measurement is evaluable at the patient's body together with a blood analysis in an extracorporeal circuit of the dialysis device.

6. Dialysis arrangement according to one of the preceding claims, **characterized in that** the patient supporting device comprises an optical measuring device for the preferable multi-dimensional, in particular three-dimensional, detection of the patient's measures.

7. Dialysis arrangement according to one of the preceding claims, **characterized in that** the patient supporting device comprises an arrangement for detecting and/or calculating the hydrostatic pressure, at least using position-sensing parameters of the patient supporting device, in particular an inclination of the backrest, leg rest, and/or footrest, and/or wherein the patient supporting device comprises an arrangement for detecting the positions of armrests of the patient supporting device.

8. Dialysis arrangement according to one of the preceding claims, **characterized in that** the patient supporting device has a motorized adjusting device preferably of at least a backrest, leg rest, and/or footrest, wherein the motorized adjusting device is preferably controllable by the dialysis device.

9. Dialysis arrangement according to one of the preceding claims, **characterized in that** the patient supporting device has a device for determining the patient's identity, preferably with a fingerprint sensor.

## Revendications

1. Appareil de dialyse pour la commande et/ou la régulation d'un dispositif de dialyse, présentant :
- un dispositif de dialyse (12) pour la réalisation d'un traitement de dialyse médical,
- un dispositif de support de patient (1) pour le support d'un patient avec un appareil de réalisation d'une mesure de bioimpédance au niveau d'un corps de patient, dans lequel cet appareil
- présente un dispositif (6a, 7a, 8a, 9a) pour le couplage d'un signal de mesure dans le corps de patient et
- un dispositif (6b, 7b, 8b, 9b) pour le découplage d'une grandeur de mesure électrique suscitée ainsi dans le corps de patient hors du corps de patient, et une interface pour le raccordement au dispositif de dialyse (12),
dans lequel le dispositif de support de patient (1) présente un dispositif de détermination de la grandeur de patient avec des capteurs de pression (11) et/ou champs de capteur de pression qui sont agencés dans la zone de surfaces d'appui de patient du dispositif de support de patient (1), et
- un appareil d'évaluation pour l'évaluation de la grandeur de mesure électrique découplée par l'appareil de réalisation d'une mesure de bioimpédance en tenant compte de la grandeur de patient déterminée et pour la transmission des données corrélées avec la grandeur de mesure électrique évaluée au dispositif de dialyse (12) par le biais de l'interface,
dans lequel le dispositif de dialyse (12) est conçu afin d'être commandé et/ou régulé par les données corrélées avec la grandeur de mesure électrique évaluée.

2. Appareil de dialyse selon la revendication 1, **caractérisé en ce que** l'appareil de réalisation d'une mesure de bioimpédance présente un dispositif pour la détermination du poids du patient.

3. Appareil de dialyse selon l'une quelconque des revendications précédentes, **caractérisé en ce que** l'appareil de réalisation d'une mesure de bioimpédance présente au moins deux électrodes de mise en contact du corps de patient pour le couplage du signal de mesure dans le corps de patient (= électrodes de couplage), de préférence par application d'un courant de mesure dans le corps de patient, et
au moins deux électrodes pour la mise en contact du corps de patient pour le découplage de la grandeur de mesure électrique suscitée par le signal de mesure dans le corps de patient hors du corps de patient (= électrodes de découplage), de préférence par prélèvement d'une modification de potentiel suscitée par le signal de mesure dans le corps de patient.

4. Appareil de dialyse selon l'une quelconque des revendications précédentes, **caractérisé en ce que** l'appareil de réalisation d'une mesure de bioimpédance présente une pluralité d'électrodes de couplage et d'électrodes de découplage de préférence respectivement au moins une électrode de couplage et respectivement au moins une électrode de découplage pour la mise en contact de chacun des bras et chacune des jambes du patient pour la réalisation de préférence d'une mesure de bioimpédance segmentaire.

5. Appareil de dialyse selon l'une quelconque des revendications précédentes, **caractérisé en ce que** la grandeur de mesure électrique de la mesure de bioimpédance peut être évaluée ensemble au niveau du corps de patient avec une analyse de sang dans un circuit extracorporel du dispositif de dialyse.

6. Appareil de dialyse selon l'une quelconque des revendications précédentes, **caractérisé en ce que** le dispositif de support de patient présente un dispositif de mesure optique pour la détection de préférence en plusieurs dimensions, en particulier en trois dimensions des mesures de patient.

7. Appareil de dialyse selon l'une quelconque des revendications précédentes, **caractérisé en ce que** le dispositif de support de patient présente un appareil de détection et/ou de calcul de la pression hydrostatique au moins en utilisant des paramètres de réglage de position du dispositif de support de patient, en particulier de l'inclinaison du dossier et/ou support pour les jambes et/ou repose-pied, et/ou dans lequel le dispositif de support de patient présente un appareil de détection des positions des accoudoirs du dispositif de support de patient.

8. Appareil de dialyse selon l'une quelconque des revendications précédentes, **caractérisé en ce que** le dispositif de support de patient présente un dispositif de réglage motorisé de préférence d'au moins le dossier et/ou support pour les jambes et/ou repose-pied, dans lequel le dispositif de réglage motorisé peut être commandé de préférence par le dispositif de dialyse.

9. Appareil de dialyse selon l'une quelconque des revendications précédentes, **caractérisé en ce que** le dispositif de support de patient présente un dispositif de constatation de l'identité de patient, de préférence avec un capteur d'empreinte digitale.
